# EUROPÄISCHE PATENTSCHRIFT

(11) **EP 2 846 731 B1**
(45) Veröffentlichungstag und Bekanntmachung des Hinweises auf die Patenterteilung: **04.07.2018**
(21) Anmeldenummer: 13721667.7
(22) Anmeldetag: 03.05.2013
(51) Int. Cl.: A61F 2/07, D03D 3/02, A61B 17/32

(54) **INTRALUMINALE GEFÄSSPROTHESE MIT IN-SITU-FENESTRIERUNG**
INTRALUMINAL VASCULAR PROSTHESIS HAVING IN-SITU FENESTRATION
PROTHÈSE VASCULAIRE INTRALUMINALE À FENESTRATION IN SITU

(30) Priorität: 07.05.2012 DE 102012103987
(43) Veröffentlichungstag der Anmeldung: 18.03.2015
(73) Patentinhaber: JOTEC GmbH, 72379 Hechingen (DE)
(72) Erfinder: GLUECK, Sascha, 72224 Ebhausen (DE); HOFFMANN, Wolf, 72770 Reutlingen (DE); WOERNE, Christian, 73760 Ostfildern (DE)
(74) Vertreter: Witte, Weller & Partner Patentanwälte mbB
(86) Internationale Anmeldenummer: PCT/EP2013/059273
(87) Internationale Veröffentlichungsnummer: WO 2013/167492

(56) Entgegenhaltungen:
- WO-A1-2010/024849
- WO-A1-2010/024880
- US-A1- 2005 240 261

## Beschreibung

Die vorliegende Erfindung betrifft eine intraluminale Gefäßprothese zur Implantation in ein Blutgefäß eines Patienten, mit in ihrer Längsrichtung hintereinander angeordneten Ringen aus mäanderförmig umlaufenden Stützen und einem an den Ringen befestigten und diese verbindenden Prothesenmaterial, wobei das Prothesenmaterial einen hohlzylindrischen Körper mit umfänglich geschlossenem Mantel bildet, und wobei das Prothesenmaterial eine Gewebestruktur aufweist, welche aus mindestens zwei Fadensystemen, nämlich mindestens einem Kettfaden und mindestens einem Schussfaden, gebildet ist.

Insbesondere betrifft die vorliegende Erfindung intraluminale Gefäßprothesen, die zur in-situ-Ausbildung von Fenestrierungen im Prothesenmaterial geeignet sind, sowie Verfahren zur Ausbildung von Fenestrierungen in intraluminalen Gefäßprothesen.

Allgemein ist es bekannt, intraluminale Gefäßprothesen, die auch als endovaskuläre Gefäßstents/-stentgrafts bezeichnet werden, zur Behandlung von Aneurysmen in Arterien zu implantieren. Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen oder, wie bei dem sog. falschen Aneurysma bzw. der sog. Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einem Ruptur der Arterie führen mit der Folge, dass der Patient innerlich verblutet.

Zur Behandlung von Aneurysmen ist es bereits bekannt, die betroffene Arterie durch Implantation eines Stents/Stentgrafts zu stabilisieren, um eine Ruptur des Gefäßes zu vermeiden.

Die zur Behandlung von Aneurysmen verwendeten Stents/Stengrafts bestehen dabei im Allgemeinen aus einem röhrchenförmigen Metallrahmen, dessen Mantelfläche mit einer Textil- oder Polymerfolie abgedeckt ist, so dass sich ein hohlzylindrischer Körper ergibt. Zur Implantation wird der Stent - bspw. mittels einer diesen umgebenden und komprimierenden Hülle - radial zusammengedrückt, so dass sich seine Querschnittsfläche deutlich verringert. Der Stent/Stentgraft wird dann mit Hilfe eines Einführsystems in den Bereich des Aneurysmas gebracht, wo der Stent freigesetzt wird. Aufgrund der Federwirkung des Metallrahmens expandiert der Stent wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch den Stent/Stentgraft, wodurch eine weitere Belastung der Aussackung verhindert wird.

Die Expansion des Metallrahmens kann einerseits durch die Verwendung von selbstexpandierendem Metall, wie bspw. Nitinol, bewirkt werden, oder aber durch Einsatz eines Dilatations-Ballons, der von innen in den Metallrahmen eingeführt wird und durch dessen Dilatation auch der Metallrahmen expandiert wird.

Oftmals zweigen an der Stelle des Gefäßes, an welcher ein solcher Stent/Stentgraft bzw. eine solche Gefäßprothese eingeführt werden soll, SeitenBlutgefäße ab, weshalb bei Einbringung des Gefäßimplantats dann die Gefahr besteht, dass diese Seitengefäße durch die Gefäßprothese im Hauptgefäß bzw. durch das blutdichte Prothesenmaterial von der Blutzufuhr abgeschnitten werden. Daher weisen Gefäßprothesen in diesen Bereichen oftmals Öffnungen, sog. "Fenestrierungen", im Mantel- bzw. Prothesenmaterial auf, um das durch die Gefäßprothese fließende Blut durch diese Öffnungen auch in die vom Gefäß abzweigenden Seiten-Gefäße zu lenken. Dadurch wird auch eine Blutversorgung der Körperbereiche, die von den Seite-Gefäßen versorgt werden, gewährleistete.

Die an solchen Abzweigungs-Bereichen einzubringende Gefäßimplantate weisen in vielen Fällen selber noch vom Gefäßprothesen-Grundkörper abzweigende Seitenäste auf, die in de Seiten-Gefäße hineinreichen. Dadurch wird zusätzlich sicher gestellt, dass auch die Seiten-Gefäße mit Blut versorgt werden.

So ist bspw. aus der US 2008/0312732 A1 ein Stentgraft bekannt, der eine röhrenförmige Wand mit zumindest einer Fenestrierung und einer peripheren Verstärkung um zumindest einen Teil der Fenestrierung herum aufweist. Der Stentgraft kann ferner röhrenförmige "Extensionen" aufweisen, also Seitenärmchen, die sich von dem Grundkörper des Stentgrafts erstrecken und die mit dem Stentgraft über die Fenestrierung in Fluid-Kommunikation stehen.

Ferner ist aus der WO 2009/064672 A2 eine Vorrichtung und ein Verfahren zur in-situ-Stengraft-Fenestrierung bekannt, bei welchem der Haupt-Stentgraft in situ, d.h. also nach im Gefäß erfolgter Positionierung, mit einer Nadel penetriert wird, um ein Nadelloch im Graft-Material zu bilden. Anschließend wird eine Dilator-Anordnung durch das Nadelloch geschoben, um das Nadelloch zu erweitern. Aus der WO2010/024880 A1 ist eine intraluminale Gefäßprothese mit einem gewebten Prothesenmaterial bekannt, in welchem Fenestrierungs-Bereiche vorgesehen sind.

Nachteile der im Stand der Technik bekannten Stentgrafts für die in-situ-Fenestrierung bestehen darin, dass sie sehr korrekt in Bezug auf die abzweigenden Gefäße positioniert werden müssen, und insbesondere darin, dass das Graft-Material an vorgesehenen Positionen zerrissen bzw. verletzt wird, um die Fenestrierungen zu bilden. Dadurch besteht die Gefahr des weiteren Ausreißens des Graft-Materials, so dass sich die Fenestrierung unkontrolliert erweitert, wodurch es als Folge wiederum zu einem unkontrollierten Ausfließen des Blutes in diesem Bereich der Gefäßprothese kommen kann. Daher ist es bei den im Stand der Technik bekannten Gefäßimplantaten für die in-situ-Fenestrierung meist notwendig, ein auf den jeweiligen Patienten bzw. dessen Gefäß genau zugeschnittenes Gefäßimplantat bereitzustellen, wenn eine erfolgreiche Behandlung erreicht werden soll.

Aufgabe der vorliegenden Erfindung ist es daher, eine intraluminale Gefäßprothese bzw. einen Stentgraft bereitzustellen, mit dem die oben geschilderten Nachteile überwunden werden können, und mit welchen Gefäßimplantate bereitgestellt werden können, die flexibel, d.h. nicht maßgeschneidert eingesetzt werden können.

Die Erfindung ist durch die Ansprüche definiert. Erfindungsgemäß wird diese Aufgabe durch eine intraluminale Gefäßprothese erreicht, die in Längsrichtung hintereinander angeordnete Ringe aus mäanderförmig umlaufenden Stützen und ein an den Ringen befestigtes und diese verbindende Prothesenmaterial aufweist, welches Prothesenmaterial einen hohlzylindrischen Grundkörper mit umfänglich geschlossenem Mantel bildet, und wobei das Prothesenmaterial eine Gewebestruktur aus mindestens jeweils einem, miteinander verwebten Kettfaden und Schussfaden aufweist, und wobei zumindest zwischen zwei hintereinander angeordneten Ringen der Gefäßprothese im Prothesenmaterial zumindest ein definierter Fenestrierungs-Bereich für die Ausbildung von zumindest einer Öffnung für von den hohlzylindrischen Grundkörper abgehende Seitenäste vorgesehen ist, wobei der Fenestrierungs-Bereich eine zum übrigen Prothesenmaterial unterschiedliche Gewebestruktur aus einer Vielzahl von nicht miteinander verwebten Kettfäden und Schussfäden aufweist, bzw. aus dieser besteht.

Die erfindungsgemäße Prothese kann benutzt werden in einem Verfahren zur Fenestrierung einer intraluminalen Gefäßprothese, wobei das Verfahren die Schritte des Bereitstellen eines intraluminalen Gefäßprothese wie zuvor beschrieben umfasst, sowie den Schritt des Durchdringens des Fenestrierungs-Bereiches der intraluminalen Gefäßprothese mit einem Erweiterungselement zur Ausbildung von zumindest einer Öffnung im Fenestrierungs-Bereich, wobei das Prothesenmaterial im Bereich der zu bildenden Öffnung durch das Erweiterungselement verdrängt wird.

Die der Erfindung zugrundeliegende Aufgabe wird auf diese Weise vollkommen gelöst.

Mit der erfindungsgemäßen Gefäßprothese wird dem Anwender der Gefäßprothese die Möglichkeit geschaffen, diese ohne Rücksicht auf bereits festgelegt Fenestrierungen bzw. Fenestrierungs-Bereiche in der Gefäßprothese zügig in einem Gefäß freizusetzen, wobei ggf. lediglich darauf geachtet werden muss, dass dann, wenn lediglich ein Fenestrierungs-Bereich vorgesehen ist, sich dieser im Bereich der von dem Hauptgefäß abgehenden Seiten-Gefäße befindet. Der Anwender kann nun in diesem Fenestrierungs-Bereich das Prothesenmaterial, bspw. mit Hilfe eines Dilators oder eines Führungsdrahtes oder eines sonstigen geeigneten Elements derart durchdringen, dass das Gewebe nicht zerrissen, zerstört, durchlöchert oder in sonstiger Weise verletzt wird, sondern die Fäden der Gewebestruktur von dem einzusetzenden Element verdrängt werden, so dass sich sozusagen eine große Masche bildet, die die Öffnung oder Fenestrierung für das abgehende Seitengefäß darstellt. Ermöglicht wird dies durch die besondere Gewebestruktur in dem Fenestrierungs-Bereich, nämlich derart, dass die Kettfäden und die Schussfäden, die im übrigen Prothesenmaterial miteinander verwebt sind, im Fenestrierungs-Bereich nicht miteinander verwebt sind. Dies hat zur Folge, dass im Fenestrierungs-Bereich Kettfäden und Schussfäden lediglich übereinander liegen, also praktisch "schichtweise" angeordnet sind. Dies wird durch ein besonderes Webverfahren erreicht, mithilfe dessen erreicht wird, dass gezielt und ausschließlich im Fenestrierungs-Bereich die Fadensysteme - also Kett- und Schussfaden - nicht miteinander verwebt werden.

Mit den Begriffe "Kettfäden" und "Schussfäden" sind vorliegend - wie auch üblicherweise im die Erfindung betreffenden Gebiet - die bei Geweben üblicherweise verwendeten beiden Fadensysteme gemeint; zur manuellen oder maschinellen Herstellung gewebter textiler Erzeugnisse werden mindestens zwei Fadensysteme rechtwinklig oder nahezu rechtwinklig verkreuzt. Dabei werden üblicherweise die Fäden in Längsrichtung als Kette oder Kettfäden bezeichnet, und die Querfäden Schuss oder Schussfäden. Die Fäden werden durch Fadenkreuzung verbunden, was nicht bedeutet, dass die Fäden kreuzend aufeinander liegen, sondern dass Fäden in einem bestimmten Rhythmus über und unter den querliegenden Fäden durchgeführt werden.

Üblicherweise werden die Garne, die zur Herstellung von intraluminalen Gefäßprothesen eingesetzt werden, in verschiedenen Richtungen zusammengewebt, bspw. derart, dass ein Satz Kettenfäden in Längsrichtung parallel zu den Webkanten verläuft und dadurch die Breite des gewebten Produktes darstellt, und dass ein Satz Schussfäden dann von Webkante zu Webkante unter einem rechten Winkel zu den Kettenfäden in diese gewebt werden.

Die hohlzylindrische Form der Gefäßprothese wird dann üblicherweise dadurch erreicht, dass das gewebte Prothesenmaterial vernäht und ggf. zugeschnitten wird. So werden bspw. auch die mäanderförmig umlaufenden Ringe jeweils mit dem Prothesenmaterial vernäht, zur Ausbildung der Röhrenform der Prothese.

Durch die im Fenestrierungs-Bereich nicht miteinander verwebten Kett- und Schussfäden wird in diesen Bereichen eine Struktur geschaffen, die leicht mit einem Erweiterungselement - von innen oder von außen der Gefäßprothese - durchdrungen werden kann, wobei beim Durchdringen lediglich die Fäden in diesem Bereich durch das Erweiterungselement verdrängt werden und die Fäden nicht zerrissen oder Durchschnitten werden. Eine Verdrängung der Fäden zur Ausbildung einer Öffnung bzw. Fenestrierung ist in den anderen, nicht mit solchen erfindungsgemäßen Fenestrierungs-Bereichen versehenen Prothesenmaterial-Bereichen nicht möglich, da hier die Gewebestruktur durch die Verwebung der Fadensysteme zu dicht ist.

Vorteilhafterweise kann die Gewebestruktur des Prothesenmaterials im Fenestrierungs-Bereich durch eine integrierte Webung hergestellt und das Prothesenmaterial einstückig gewebt und ausgebildet werden. Hierzu ist der Webeprozess derart ausgebildet bzw. mittels entsprechender Software programmiert, dass im Fenestrierungs-Bereich Kettfäden und Schussfäden bzw. Kettfaden und Schussfaden nicht miteinander verwebt werden.

Vorliegend werden die Begriffe "Öffnung" und "Fenestrierung" auch synonym verwendet.

Die in einem Abstand hintereinander angeordneten Ringe aus mäanderförmig umlaufenden Stützen stellen Stents bzw. Stent-Ringe dar.

Gemäß einer Ausführungsform kann auch vorgesehen sein, dass die Fenestrierungen durch zusätzliche Verstärkungselemente umrandet sind, welche bspw. durch Kleben oder Vernähen um den äußeren Rand des Fenestrierungs-Bereichs aufgebracht werden, um ein weiteres Ein-/Ausreißen des den Fenestrierungs-Bereich umgebenden Prothesenmaterials zu verhindern. Erfindungsgemäß bilden diese Verstärkungselemente damit also eine Ausrissgrenze, mit der verhindert wird, dass die durch die Verdrängung der Fäden gebildeten größeren Maschen bzw. Öffnungen nicht ausreißen, sondern in der vorgegebenen Dimension bzw. Grenze verbleiben. Die Materialien der Verstärkungselemente - dies können bspw. Textilfäden und/oder Klebstoffe sein - sind bspw. Polyester, Polymerschaum, UV-härtende Polymere.

Die erfindungsgemäße Gefäßprothese kann insgesamt zwischen einem und neun Fenestrierungs-Bereiche, vorzugsweise, eins, zwei, drei, vier, fünf, sechs, sieben oder acht, Fenestrierungs-Bereiche, aufweisen.

Diese Ausführungsform hat den Vorteil, dass nicht nur ein von der Gefäßprothese abgehender Seitenast über eine Fenestrierungs-Bereich angebracht werden kann, sondern mehrere, vorzugsweise so viele wie Fenestrierungs-Bereich im Prothesenmaterial vorgesehen sind.

Gemäß einer Ausführungsform der erfindungsgemäßen Gefäßprothese weist diese einen Bereich auf, der "ungestentet" ist, d.h. in diesem Gefäßprothesen-Bereich befinden sich keine hintereinander angeordneten Stent-Ringe, sondern nur Prothesenmaterial. Dieser Bereich wird aber - ebenso wie die "gestenteten" Bereiche durch zwei hintereinander angeordnete Ringe begrenzt, allerdings ist der Abstand der Ringe größer als im gestenteten Bereich/in den gestenteten Bereichen. Daher wir vorliegend auch unter den gestenteten Bereichen diejenigen Bereiche verstanden, bei welchen sich die Stent-Ringe in engem bzw. keinen Abstand voneinander befinden; es versteht sich, dass auch im gestenteten Bereich Prothesenmaterial vorliegt, das frei von Stent-Material ist, was in der Natur der mäanderförmig umlaufenden Ringe/Stützen liegt.

Gemäß einer Ausführungsform der erfindungsgemäßen Gefäßprothese sind daher entweder nur im gestenteten Bereich oder nur im ungestenteten Bereich oder sowohl im gestenteten Bereich Fenestrierungs-Bereiche vorgesehen. In den ersten beiden Fällen liegt zumindest ein Fenestrierungs-Bereich vor, im letzteren Fall mindestens zwei. Dem Fachmann wird ausgehend von der Offenbarung dieser Erfindung klar sein, dass die Fenestrierungs-Bereiche im Umfang des Prothesenmaterials der Gefäßprothese je nach bzw. mit Rücksicht auf die Ausbildung bzw. Lage der Gefäße ausgebildet sind und ggf. über die Länge der Gefäßprothese verteilt sind.

Entsprechend kann die erfindungsgemäße Gefäßprothese daher bspw. im Aortenbogen oder im thorakalen oder iliakalen Bereich, mithin also vorzugsweise in allen Gefäßbereichen, die einer Unterstützung bedürfen, und von denen im zu behandelnden Abschnitt Seitenblutgefäße abzweigen, eingesetzt werden.

Ein Fenestrierungs-Bereich im gestenteten Bereich ist derart vorgesehen, dass er bspw. zwischen den Bögen der mäanderförmig umlaufenden Ringe im Prothesenmaterial vorgesehen ist. Gemäß einer Ausführungsform der erfindungsgemäßen Gefäßprothese ist bevorzugt, wenn zwischen einem und acht, also ein, zwei, drei, vier, fünf sechs sieben oder acht, Fenestrierungs-Bereiche im ungestenteten oder im gestenteten Bereich vorgesehen sind.

Gemäß einer Weiterbildung der erfindungsgemäßen intraluminalen Gefäßprothese besitzt der zumindest eine Fenestrierungs-Bereich eine Form, die ausgewählt ist aus rund, kreisförmig oder oval.

Dabei ist dem Fachmann klar, dass die Form nicht exakt kreisförmig sein muss, um die Zwecke der vorliegenden Erfindung zu erfüllen, sondern auch "im Wesentlichen" kreisförmig, also auch alle Formen, die leichte Abweichungen von der kreisförmigen Form aufweisen, die aber immer noch geeignet sind, nach deren Durchdringen eine röhrenförmige Seiten-Prothese aufzunehmen.

Es versteht sich, dass dem Fachmann anhand der vorliegend bereitgestellten Lehre klar sein wird, dass die erfindungsgemäße Gefäßprothese an verschiedenen Stellen über ihre gesamte Länge und über den gesamten Umfang hinweg Fenestrierungs-Bereiche aufweisen kann, die in Abhängigkeit des zu behandelnden Gefäßes, bzw. in Abhängigkeit von vorliegenden abgehenden Gefäßen dimensioniert werden können.

Gemäß einer Ausführungsform der erfindungsgemäßen Gefäßprothese besitzt der zumindest eine Fenestrierungs-Bereich einen Durchmesser von ca. zwischen 2 bis 15 mm. Es versteht sich, dass auch die Durchmesser der Fenestrierungs-Bereiche mit Rücksicht auf den jeweils abgehenden Seitenast der Prothese bzw. auf das abgehende Blutgefäß dimensioniert sein werden.

Bei einem Vorliegen mehrerer Fenestrierungs-Bereiche können diese entsprechend in einer Ausführungsform der erfindungsgemäßen Gefäßprothese die gleichen oder unterschiedliche Durchmesser aufweisen.

Gemäß einer bevorzugten Ausführungsform weist die intraluminale Gefäßprothese einen Durchmesser von zwischen 5 mm und 70 mm auf, wobei bevorzugt ist, dass der Durchmesser zwischen 10 und 50 mm liegt.

Dem Fachmann wird allerdings klar sein, dass auch andere Bereiche möglich sind, und dass der letztendlich gewählte Durchmesser in Abhängigkeit des zu behandelnden Gefäßes gewählt werden wird.

Geeignete Materialien für die intraluminale Gefäßprothese sind für die hintereinander angeordneten Ringe ein selbstexpandierendes Material, beispielsweise Nitinol, sowie für das Prothesenmaterial insbesondere Polyester, Polypropylen, Polyurethan, Polytetrafluoräthylen oder ultrahochmolekulargewichtiges Polyethylen (UHMPE), oder Mischungen davon, wobei Polyestergewebe bevorzugt ist, bzw. Garne aus diesen Materialien. Dem Fachmann wird auch bezüglich der Materialien und unter Bezugsnahme auf die hierin offenbarte Lehre klar sein, dass auch andere, im Stand der Technik bekannte Materialien zum Einsatz kommen können, mithin also alle Materialien, die zur Ausbildung einer Gewebestruktur geeignet sind, und gleichzeitig dehnbare Maschen aufweisen.

Entsprechend betrifft die vorliegende Erfindung auch eine intraluminale Gefäßprothese, die neben dem hohlzylindrischen Grundkörper noch zumindest einen weiteren hohlzylindrischen Seitenkörper umfasst, welcher in die im Fenestrierungs-Bereich auszubildende zumindest eine Öffnung einbringbar ist.

Wie bereits einleitend erwähnt, können in die im Fenestrierungs-Bereich auszubildenden Öffnungen Seitenäste eingebracht werden, nachdem die intraluminale Gefäßprothese in das zu behandelnde Gefäß eingebracht und die Öffnungen ausgebildet wurden. Diese Seitenkörper werden durch die intraluminale Gefäßprothese und durch die Öffnung in das abgehende Seitengefäß eingeführt, um diesem den Blutfluss, der durch die intraluminale Gefäßprothese geleitet wird, aufrechtzuerhalten. Dabei ist bevorzugt, wenn diese zweiten Gefäßprothesen Metall-Stents ohne Prothesenmaterial oder aber ebenfalls Stentgrafts mit Prothesenmaterial darstellen.

Die erfindungsgemäße Gefäßprothese kann in einer Weiterbildung ferner entweder am hohlzylindrischen Grundkörper, oder an dem/den jeweiligen Fenestrierungs-Bereich(en), und/oder an dem Seitenast/den Seitenästen bspw. röntgendichte Marker aufweisen, die das Einführen und Positionieren der Gefäßprothese erleichtern. Wenn bei der erfindungsgemäßen Prothese mehrere Fenestrierungs-Bereiche vorgesehen sind, so können diese bspw. unterschiedliche Marker aufweisen.

Wie weiter oben erwähnt, kann die erfindungsgemäße Prothese auch verwendet werden in einem Verfahren zur Fenestrierung einer intraluminalen Gefäßprothese, das die folgenden Schritte aufweist:
- Bereitstellen einer erfindungsgemäßen intraluminalen Gefäßprothese,
- Durchdringen des zumindest einen Fenestrierungs-Bereiches der intraluminalen Gefäßprothese mit einem Erweiterungselement, insbesondere einer Dilatorspitze oder einer Führungsdrahtspitze, zur Ausbildung von zumindest einer Öffnung im Fenestrierungs-Bereich, wobei das Prothesenmaterial im Bereich der zu bildenden Öffnung durch das Erweiterungselement verdrängt wird.

Dieses Verfahren bietet den Vorteil, dass das Prothesenmaterial der Gefäßprothese nicht verletzt, zerstört oder zerrissen werden muss, um Öffnungen in dem Fenestrierungs-Bereich auszubilden. Vielmehr können durch das Erweiterungselement die im Fenestrierungs-Bereich nicht miteinander verwebten Ketten- und Schussfäden einfach jeweils rechts und links von dem Erweiterungselement zur Seite gedrängt werden, zur Ausbildung der Öffnung. Dadurch wird das Prothesenmaterial weder zerrissen noch durchstochen, weshalb die Gefahr eines Einreißens des Prothesenmaterials in Bereiche außerhalb des Fenestrierungs-Bereiches vermieden wird.

Dabei wird vorliegend unter einem "Erweiterungselement" jedes Mittel verstanden, mit Hilfe dessen in dem Fenestrierungs-Bereich eine Öffnung/Fenestrierung geschaffen werden kann, ohne das Prothesenmaterial zu verletzen oder zu zerreißen. Geeignete Erweiterungselemente sind dabei bspw. Ein Katheter, Dilator oder ein Führungsdraht, bzw. die Katheter-, Dilatoren- oder Führungsdrahtspitze.

Katheter, Dilatoren und Führungsdrähte sind im Stand der Technik hinreichend bekannt und werden bereits seit längerem im Zusammenhang mit Gefäßprothesen eingesetzt, so dass dem Fachmann klar sein wird, welche Mittel er erfindungsgemäß einsetzen kann.

Ferner ist offenbart ein Verfahren zur Einbringung einer intraluminalen Gefäßprothese, wobei das Verfahren die folgenden Schritte aufweist:
- Einbringen und Freisetzen einer ersten erfindungsgemäßen intraluminalen Gefäßprothese in ein Blutgefäß eines Patienten,
- Ausbilden von zumindest einer Öffnung im Fenestrierungs-Bereich des hohlzylindrischen Grundkörpers durch Verdrängen des Prothesenmaterials im Fenestrierungs-Bereich des Prothesenmaterials,
- ggf. Einbringen einer zweiten Gefäßprothese durch die Öffnung im Fenestrierungs-Bereich des hohlzylindrischen Grundkörpers zur Ausbildung von zumindest einem Seitenast der intraluminalen Gefäßprothese.

Mit diesem Verfahren kann erstmals die Möglichkeit geschaffen werden, eine Gefäßprothese in ein Gefäß zum Zwecke der Ausbildung von zumindest einer Fenestrierung/Öffnung einzuführen, ohne dass die genaue Rotations-Position der Gefäßprothese in dem Gefäß berücksichtigt werden müsste. Der chirurgische Eingriff wird dadurch wesentlich erleichtert und beschleunigt.

Um die erfindungsgemäße Gefäßprothese in das zu behandelnde Gefäß einzubringen, können im Stand der Technik bekannte oder übliche Einführsysteme mit Katheter, Rückzugshülle und Pusher eingesetzt werden. Auch diese sind dem Fachmann bekannt.

Typische Anwendungsgebiete sind, wie bereits weiter oben beschrieben, der Einsatz im Aortenbogen, im thorakalen und im iliakalen Bereich.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung eines Ausschnitts einer Ausführungsform der erfindungsgemäßen intraluminalen Gefäßprothese;
- Fig. 2: eine schematische Darstellung eines Ausschnitts einer weiteren Ausführungsform der erfindungsgemäßen Gefäßprothese;
- Fig.3: eine schematische Darstellung eines Ausschnitts einer weiteren Ausführungsform der erfindungsgemäßen Gefäßprothese, mit eingeführtem Seitenast;
- Fig. 4a: zwei in Blutgefäße eingebrachte Ausführungsbeispiele der intraluminalen Gefäßprothese, wie in Fig. 3 gezeigt, wobei diese Ausführungsbeispiele im Bereich der Aorten-Bifurkation eingeführt sind;
- Fig. 4b: eine schematische Darstellung einer erfindungsgemäßen intraluminalen Gefäßprothese, die mit Seitenästen in den Aortenbogen eingebracht ist;
- Fig. 4c: eine schematische Darstellung einer noch weiteren Ausführungsform der intraluminalen Gefäßprothese, eingebracht in ein weiteres Gefäß eines Patienten, mit vier abgehenden Seitenästen der Gefäßprothese; und
- Fig. 4d: eine schematische Darstellung einer weiteren Ausführungsform der erfindungsgemäßen Gefäßprothese, eingebracht im Aorto-bi-iliakalen Bereich.

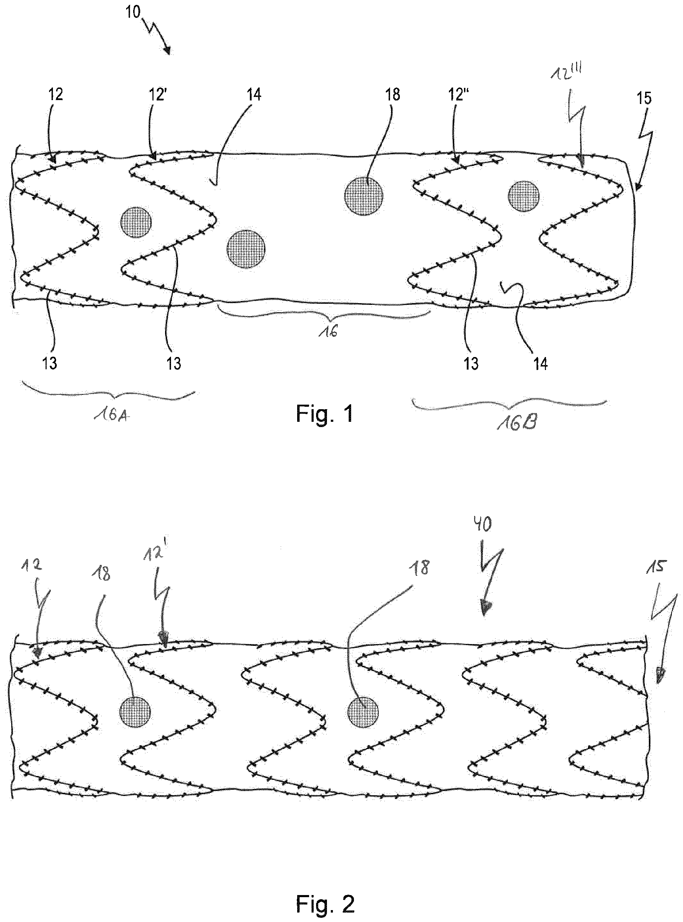

In Fig. 1 ist mit 10 insgesamt eine intraluminale Gefäßprothese bezeichnet, mit in ihrer Längsrichtung hintereinander angeordneten Ringen 12, 12' aus mäanderförmig umlaufenden Stützen 13 und einem an den Ringen 12, 12' befestigten und diese verbindenden Prothesenmaterial 14. Zwischen dem Ring 12' und 12" besitzt die Gefäßprothese 10 einen ungestenteten Bereich 16, der in dem in Fig. 1 gezeigten Ausführungsbeispiel durch eine umgreifende Klammer angezeigt ist. In diesem Bereich sind keine Ringe 12 vorgesehen, die unmittelbar oder in kleinem Abstand voneinander angeordnet sind, wie es der Fall ist bei den Bereichen 16A und 16B ist, die rechts und links von dem ungestenteten Bereich 16 liegen.

Fig. 2 zeigt die in Fig. 1 gezeigte eine andere Ausführungsform der erfindungsgemäßen intraluminalen Gefäßprothese 60, wobei hier gleiche Merkmale wie in Fig. 1 mit den gleichen Bezugszeichen versehen sind; diese Ausführungsform weist keinen ungestenteten Bereich auf, sondern regelmäßig hintereinander angeordnete Stent-Ringe 12, 12', wobei zwischen diesen Ringen das Prothesenmaterial frei von Stent-Ringen 12, 12' ist.

Die in Fig. 1 und 2 gezeigten Ausführungsformen der Gefäßprothese 10; 60 weisen beide einen hohlzylindrischen Grundkörper 15 auf, sowie Fenestrierungs-Bereiche 18, die in den dargestellten Ausführungsformen kreisförmig bzw. rund sind.

Wie Fig. 1 zu entnehmen ist, weist die dort dargestellte Ausführungsform sowohl im ungestenteten Bereich 16 als auch in den gestenteten Bereichen 16A und 16B Fenestrierungs-Bereiche 18 auf, wobei die Fenestrierungs-Bereiche 18 im ungestenteten Bereich 16 einen größeren Durchmesser haben.

Die Fenestrierungs-Bereiche 18 können, wie weiter oben in der allgemeinen Beschreibung der Erfindung dargelegt, auf dem jeweiligen Umfang verteilt vorliegen, unterschiedliche Durchmesser aufweisen, sowie nur im gestenteten Bereich 16A oder 16B als auch nur im ungestenteten Bereich 16 vorliegen. Die in den Figuren dargestellten Figuren stellen lediglich beispielhafte Ausführungen dar, die der besseren Veranschaulichung der Erfindung dienen.

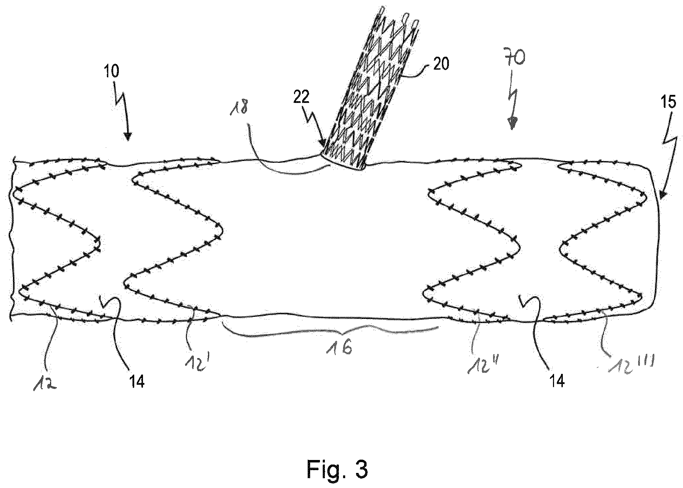

In Fig. 3 ist eine weitere Ausführungsform einer erfindungsgemäßen Gefäßprothese 40 gezeigt, wobei hier durch den hohlzylindrischen Grundkörper 15 ein Seitenast oder eine Seitenprothese 20 vorgesehen ist, der durch eine im Fenestrierungs-Bereich 18 vorgesehene Öffnung 22 durchgeschoben wurde. Bei der in Fig. 3 gezeigten Ausführungsform der Seitenprothese 20 handelt es sich um einen Stent, der bei dieser Ausführungsform kein Prothesenmaterial 14 aufweist. Es versteht sich, dass auch andere Ausführungsformen für Seitenäste/-prothesen 20 durch die im Fenestrierungs-Bereich 18 zu bildenden Öffnung 22 hindurchgeschoben werden können, deren Ausgestaltungen und Dimensionen dem Fachmann ausgehend von der hierin offenbarten Lehre klar sein werden.

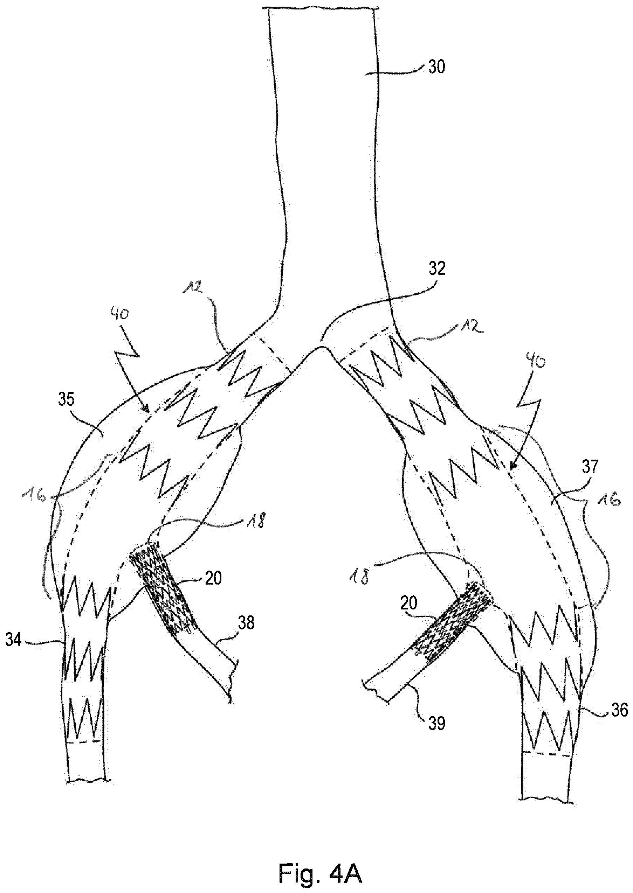

In den Fig. 4a bis 4d werden nun Ausführungsbeispiele für in Gefäße eingeführte erfindungsgemäße intraluminale Gefäßprothesen beschrieben.

In Fig. 4a ist gezeigt wie die in Fig. 3 dargestellte Ausführungsform der erfindungsgemäßen intraluminalen Gefäßprothese 70 in der Aorten-Bifurkation positioniert ist. Dabei ist mit 30 insgesamt die Aorta bezeichnet, wobei das Bezugszeichen 32 die Aortengabel (Bifurcatio aortae) angibt. An der Aortengabel 32 teilt sich die Aorta 30 in die beiden Arteriae iliacae 34 und 36, die jeweils in das linke und rechte Bein hinabführen. Im jeweils oberen Bereich der Arteriae iliacae 34, 36 befindet sich ein Aneurysma 35 bzw. 37, das jeweils durch Einbringung einer erfindungsgemäßen intraluminalen Gefäßprothese 10 überbrückt worden ist.

Wie Fig. 4a zu entnehmen ist, wurde die intraluminale Gefäßprothese 70 jeweils derart in die Arteria iliaca 34, 36 eingeführt bzw. positioniert und freigesetzt, dass der Fenestrierungs-Bereich 18 im Bereich eines abgehenden Gefäßes 38 bzw. 39 zu liegen kommt. Ferner weist die erfindungsgemäße intraluminale Gefäßprothese 70 jeweils eine durch den Fenestrierungs-Bereich 18 geführte Seitenprothese 20 auf, die in das Seitengefäß 38 bzw. 39 gelegt ist.

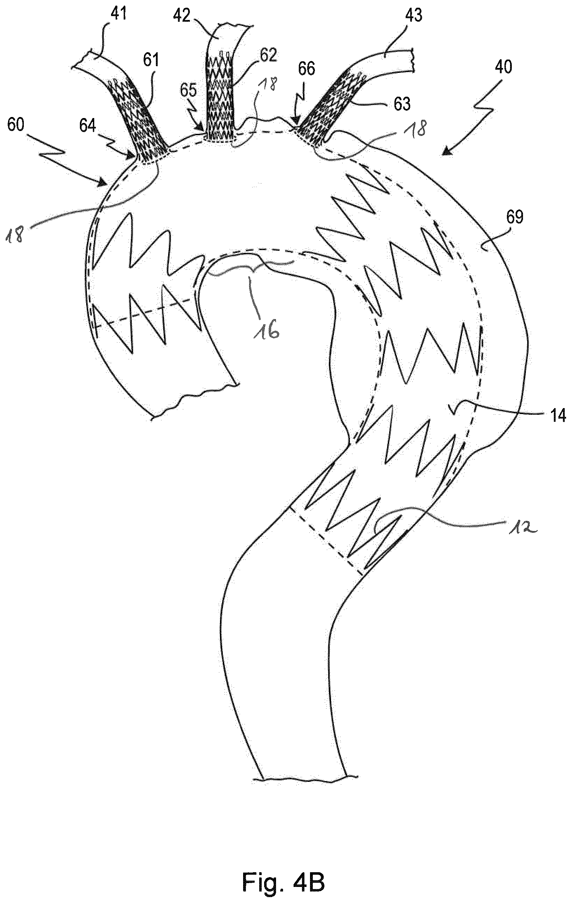

Fig. 4b zeigt eine weitere Ausführungsform 60 der erfindungsgemäßen intraluminalen Gefäßprothese, wobei bei der in Fig. 4b gezeigten Ausführungsform gleiche Merkmale wie bei den in den Fig. 1 bis 3 gezeigten Ausführungsformen mit gleichen Bezugszeichen versehen sind. Auch die Gefäßprothese 60 weist hintereinander angeordnete mäanderförmig umlaufende Ringe 12 auf, mit einem daran angebrachten Prothesenmaterial 14.

Ferner weist die in Fig. 4b gezeigte intraluminale Gefäßprothese 60 im ungestenteten Bereich 16 Fenestrierungs-Bereiche 18 auf, in welchem Öffnungen 64, 65, 66 gebildet sind, durch welche Seitenäste/-prothesen 61, 62, 63 angebracht sind. Das in Fig. 3b gezeigte Ausführungsbeispiel ist dabei im Aortenbogen 40 eingebracht, wobei mit 41, 42 und 43 die abgehenden Gefäße des Truncus brachiocephalicus, der Arteria carotis communis und der Arteria subclavia bezeichnet sind.

Auch bei der in Fig. 4b gezeigten Darstellung des Aortenbogens 40 ist mit 64 ein Aneurysma 64 bezeichnet, das durch die intraluminale Gefäßprothese 60 überbrückt wird. Es versteht sich, dass die intraluminale Gefäßprothese 60 insbesondere in diesem Bereich des Aneurysmas 64 blutdicht oder nahezu Blutdicht ist.

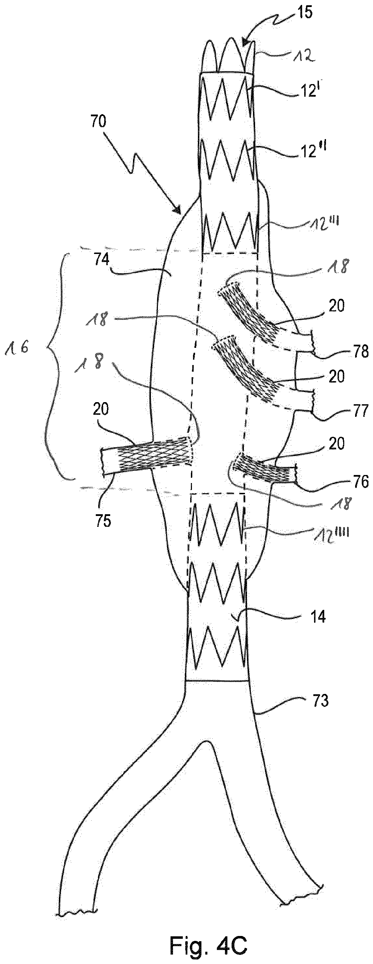

Fig. 4c zeigt schließlich eine weitere Ausführungsform 70 der erfindungsgemäßen Gefäßprothese, mit hintereinander angeordneten mäanderförmig umlaufenden Ringen 12, 12', 12", 12"', 12"", und mit Fenestrierungs-Bereichen 18 im ungestenteten Bereich 16, der von den Ringen 12'" und 12"" begrenzt wird.

Bei der in Fig. 4c gezeigten Ausführungsform 70 sind vier Seitenäste/- prothesen 20 vorgesehen, die im Bereich eines Aneurysmas 74 in abzweigenden Seitengefäße 75, 76, 77 und 78 hineinreichen.

In Fig. 4c ist gut zu erkennen, dass die drei Seitenäste 20 der Gefäßprothese 70 auf dem Umfang und der Länge der Gefäßprothese 70 verteilt bzw. versetzt vorgesehen sein können, und zwar je nach Lage der abgehenden und zur versorgenden Seitengefäße.

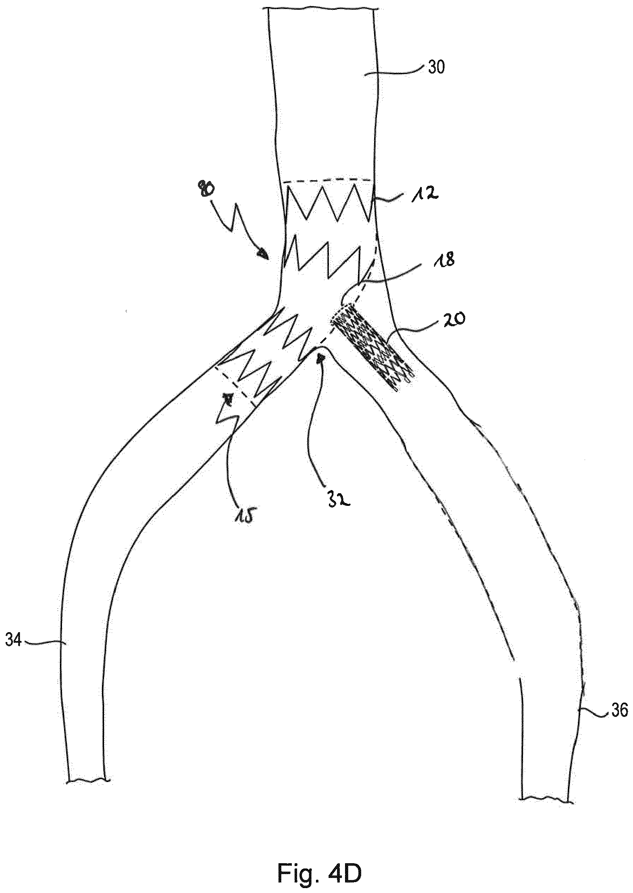

Fig. 4d zeigt schließlich eine weitere Möglichkeit für einen Einsatz einer erfindungsgemäßen Gefäßprothese 80. Diese ist - wie in Fig. 4A - wieder im Bifurkations-Bereich der Aorta eingeführt, diesmal allerdings direkt im Bereich der Bifurkation. Der Fenestrierungs-Bereich 18 ist derart in der Bifurkation 32 der Aorta 30 platziert, dass durch die Öffnung, die im Fenestrierungs-Bereich 18 gebildet wird, ein Seitenast 20 in die Arteria iliaca gelegt werden kann. Der hohlzylindrische Grundkörper 15 der Gefäßprothese 80 erstreckt sich dabei von der Arteria iliaca über die Bifurcatio Aortae in die Aorta.

Die in den Figuren 4a bis 4d gezeigten Ausführungsformen wurden wie folgt in die betreffenden Gefäße eingebracht: Zunächst wird der hohlzylindrische Grundkörper 15 im komprimierten Zustand in das zu behandelnde Gefäß eingeführt und an der erwünschten Position zur Überbrückung des Aneurysmas positioniert, und zwar derart, dass der/die Fenestrierungs-Bereich(e) im Bereich der/des vom Gefäß abgehenden Seitengefäße(s) zu liegen kommt. Nach der Freisetzung des hohlzylindrischen Körpers 15 wird mittels geeigneter Erweiterungselemente in den/dem Fenestrierungs-Bereich(en) 18 jeweils eine Öffnung/Fenestrierung geschaffen, und anschließend Seitenäste/-prothesen 20 durch diese Öffnungen vorgeschoben - je nach Möglichkeit/Notwendigkeit von innen oder von außen - zur Schaffung von Seitenästen der Gefäßprothese 10, 40, 60, 70, 80.

Die Gefäßprothese 10, 40, 60, 70 kann hierfür, entweder am hohlzylindrischen Grundkörper 15, an den jeweiligen Fenestrierungs-Bereichen 18, und/oder an dem Seitenast/den Seitenästen 20 bspw. röntgendichte Marker (nicht dargestellt) aufweisen, die das Einführen und Positionieren der Gefäßprothese 10, 40 60, 70 erleichtern. Wenn bei der erfindungsgemäßen Prothese mehrere Fenestrierungs-Bereiche vorgesehen sind, so können diese bspw. unterschiedliche Marker aufweisen.

## Patentansprüche

1. Intraluminale Gefäßprothese (10; 60; 70) zur Implantation in ein Blutgefäß, mit in ihrer Längsrichtung in einem Abstand hintereinander angeordneten Ringen (12; 12', 12", 12"') aus mäanderförmig umlaufenden Stützen (13) und einem an den Ringen (12) befestigten und diese verbindenden Prothesenmaterial (14), wobei das Prothesenmaterial (14) einen hohlzylindrischen Grundkörper (15) mit umfänglich geschlossenem Mantel bildet, und wobei das Prothesenmaterial (14) eine Gewebestruktur aus einer Vielzahl von miteinander verwebten Kettfäden und Schussfäden aufweist, wobei zumindest zwischen zwei hintereinander angeordneten Ringen (12, 12', 12", 12"') der Gefäßprothese (10; 60; 70) im Prothesenmaterial (14) zumindest ein Fenestrierungs-Bereich (18) für die Ausbildung von zumindest einer Öffnung (22) für von dem hohlzylindrischen Grundkörper (15) abgehende Seitenäste (20; 61, 62, 63) vorgesehen ist, **dadurch gekennzeichnet, dass** der Fenestrierungs-Bereich (18) eine zu dem übrigen Prothesenmaterial (14) unterschiedliche Gewebestruktur aus nicht miteinander verwebten Kettfäden und Schussfäden aufweist, derart, dass im Fenestrierungsbereich Kettfäden und Schussfäden lediglich übereinander liegen .

2. Intraluminale Gefäßprothese (10; 60; 70) nach Anspruch 1, **dadurch gekennzeichnet, dass** sie insgesamt zwischen einem und neun Fenestrierungs-Bereiche (18) aufweist.

3. Intraluminale Gefäßprothese (10; 60; 70) nach einem der Ansprüche 1 oder 2, **dadurch gekennzeichnet, dass** sie zumindest einen ungestenteten Bereich (16) aufweist, der durch zwei hintereinander angeordnete Ringen (12', 12") begrenzt ist, bei welchem Bereich (16) der Abstand der hintereinander angeordneten Ringe (12', 12") größer ist als in anderen durch hintereinander angeordnete Ringe (12, 12'; 12", 12"') begrenzten gestenteten Bereichen (16A, 16B).

4. Intraluminale Gefäßprothese (10; 60; 70) nach Anspruch 3, **dadurch gekennzeichnet, dass** zumindest ein Fenestrierungs-Bereich (18) im gestenteten Bereich (16A, 16B) und/oder zumindest ein Fenestrierungs-Bereich (18) im ungestenteten Bereich (16) vorgesehen ist.

5. Intraluminale Gefäßprothese (10; 60; 70) nach einem der Ansprüche 3 oder 4, **dadurch gekennzeichnet, dass** sie zumindest in einem gestenteten Bereich (16A, 16B) im Prothesenmaterial (14) zwischen einem und acht Fenestrierungs-Bereiche (18) aufweist.

6. Intraluminale Gefäßprothese (10; 60; 70) nach einem der Ansprüche 1 bis 5, **dadurch gekennzeichnet, dass** der zumindest eine Fenestrierungs-Bereich (18) eine runde, kreisförmige oder ovale Form besitzt.

7. Intraluminale Gefäßprothese (10; 60; 70) nach einem der Ansprüche 1 bis 6, **dadurch gekennzeichnet, dass** der zumindest eine Fenestrierungs-Bereich (18) einen Durchmesser von zwischen 2 mm bis 15 mm aufweist.

8. Intraluminale Gefäßprothese (10; 60; 70) nach einem der Ansprüche 1 bis 7, **dadurch gekennzeichnet, dass** bei Vorliegen von zumindest zwei Fenestrierungs-Bereichen (18) die Durchmesser der Fenestrierungs-Bereiche (18) gleiche oder unterschiedliche Durchmesser aufweisen.

9. Intraluminale Gefäßprothese (10; 60; 70) nach einem der Ansprüche 1 bis 8, **dadurch gekennzeichnet, dass** sie neben dem hohlzylindrischen Grundköper (15) ferner zumindest einen hohlzylindrischen Seitenkörper (20; 61, 62, 63) umfasst, welcher in die im zumindest einen Fenestrierungs-Bereich (18) auszubildende zumindest eine Öffnung (22) einbringbar ist.

## Claims

1. Intraluminal vascular prosthesis (10; 60; 70) for implantation into a blood vessel, having rings (12; 12', 12", 12"'), which are successively arranged in its longitudinal direction at a distance from one another, consisting of circumferential meandering struts (13), and a prosthesis material (14), which is fixed to the rings (12) and connects them, the prosthesis material (14) forming a hollow cylindrical body (15) with a circumferentially closed mantle and comprising a fabric structure of a plurality of warp threads and weft threads interwoven with one another, wherein in the prosthesis material (14), at least between two successively arranged rings (12, 12', 12", 12'") of the vascular prosthesis (10; 60; 70), at least one fenestration-area (18) is provided for forming of at least one opening (22) for side branches (20; 61, 62, 63) branching off from the hollow cylindrical body (15), **characterized in that** the fenestration-area (18) has a fabric structure being different from the fabric structure of the remaining prosthesis material (14), the fabric structure in the fenestration-area (18) consisting of warp threads and weft threads not being interwoven with one another, such, that in the fenestration-area (18) the warp threads and weft threads merely lie above one another.

2. The intraluminal vascular prosthesis (10; 60; 70) of claim 1, **characterized in that** it comprises between one and nine fenestration-areas (18) in total.

3. The intraluminal vascular prosthesis (10; 60; 70) of claim 1 or 2, **characterized in that** it comprises at least one nonstented area (16), which is confined by two rings (12', 12") arranged successively behind one another, in which area (16) the distance between the successively arranged rings (12', 12") is larger than in other stented areas (16A, 16B) confined by successively arranged rings (12, 12', 12", 12'").

4. The intraluminal vascular prosthesis (10; 60; 70) of claim 3, **characterized in that** in the stented area (16A, 16B) at least one fenestration-area (18) is provided and/or in the nonstented area (18) at least one fenestration-area (18) is provided.

5. The intraluminal vascular prosthesis (10; 60; 70) of one of claims 3 or 4, **characterized in that** it comprises, in at least one stented area (16A, 16B) between one and eight fenestration-areas (18) in the prosthesis material (14).

6. The intraluminal vascular prosthesis (10; 60; 70) of one of claims 1 to 5, **characterized in that** the at least one fenestration-area (18) has a round, circular or oval shape.

7. The intraluminal vascular prosthesis (10; 60; 70) of one of claims 1 to 6, **characterized in that** the at least one fenestration-area (18) has a diameter of between 2 mm and 15 mm.

8. The intraluminal vascular prosthesis (10; 60; 70) of one of claims 1 to 7, **characterized in that** the diameters of the fenestration-areas (18) are the same or different, if at least two fenestration-areas are present.

9. The intraluminal vascular prosthesis (10; 60; 70) of one of claims 1 to 8, **characterized in that** it comprises at least one hollow cylindrical side body (20; 61, 62, 63) besides the hollow cylindrical body (15), which side body (20; 61, 62, 63) is introduceable into the at least one opening (22), which is to be formed in the at least one fenestration-area (18).

## Revendications

1. Prothèse vasculaire intraluminale (10 ; 60 ; 70) destinée à être implantée dans un vaisseau sanguin, avec des anneaux (12 ; 12', 12", 12"') composés de segments périphériques sinueux (13) disposés à une certaine distance l'un derrière l'autre dans sa direction longitudinale et une matière de prothèse (14) attachée aux anneaux (12) et reliant ceux-ci, dans laquelle la matière de prothèse (14) forme un corps de base cylindrique creux (15) avec une enveloppe fermée en périphérie, et dans laquelle la matière de prothèse (14) présente une structure tissulaire composée d'une multiplicité de fils de chaîne et de fils de trame entrelacés les uns avec les autres, dans laquelle il est prévu au moins entre deux anneaux (12, 12', 12", 12"') de la prothèse vasculaire (10 ; 60 ; 70) disposés l'un derrière l'autre dans la matière de prothèse (14) au moins une région de fenestration (18) pour la formation d'au moins une ouverture (22) pour des branches latérales (20 ; 61, 62, 63) partant du corps de base cylindrique creux (15), **caractérisée en ce que** la région de fenestration (18) présente une structure tissulaire différente du reste de la matière de prothèse (14), et qui est composée de fils de chaîne et de fils de trame non entrelacés les uns avec les autres, de telle manière que dans la région de fenestration les fils de chaîne et les fils de trame reposent uniquement les uns sur les autres.

2. Prothèse vasculaire intraluminale (10 ; 60 ; 70) selon la revendication 1, **caractérisée en ce qu'**elle présente au total entre une et neuf régions(s) de fenestration (18).

3. Prothèse vasculaire intraluminale (10 ; 60 ; 70) selon une des revendications 1 ou 2, **caractérisée en ce qu'**elle présente au moins une zone non stentée (16), qui est limitée par deux anneaux (12', 12") disposés l'un derrière l'autre, région (16) dans laquelle la distance des anneaux (12', 12") disposés l'un derrière l'autre est plus grande que dans d'autres zones stentées (16A, 16B) limitées par des anneaux (12, 12' ; 12", 12"') disposés les uns derrière les autres.

4. Prothèse vasculaire intraluminale (10 ; 60 ; 70) selon la revendication 3, **caractérisée en ce qu'**il est prévu au moins une région de fenestration (18) dans la zone stentée (16A, 16B) et/ou au moins une région de fenestration (18) dans la zone non stentée (16).

5. Prothèse vasculaire intraluminale (10 ; 60 ; 70) selon une des revendications 3 ou 4, **caractérisée en ce qu'**elle présente entre une et huit région(s) de fenestration (18) au moins dans une zone stentée (16A, 16B) dans la matière de prothèse (14).

6. Prothèse vasculaire intraluminale (10 ; 60 ; 70) selon l'une quelconque des revendications 1 à 5, **caractérisée en ce que** l'au moins une région de fenestration (18) présente une forme ronde, circulaire ou ovale.

7. Prothèse vasculaire intraluminale (10; 60; 70) selon l'une quelconque des revendications 1 à 6, **caractérisée en ce que** ladite au moins une région de fenestration (18) présente un diamètre compris entre 2 mm et 15 mm.

8. Prothèse vasculaire intraluminale (10 ; 60 ; 70) selon l'une quelconque des revendications 1 à 7, **caractérisée en ce qu'**en présence d'au moins deux régions de fenestration (18), les diamètres des régions de fenestration (18) présentent des diamètres égaux ou différents.

9. Prothèse vasculaire intraluminale (10 ; 60 ; 70) selon l'une quelconque des revendications 1 à 8, **caractérisée en ce qu'**elle comprend en outre, en plus du corps de base cylindrique creux (15), au moins un corps latéral cylindrique creux (20 ; 61, 62, 63), qui peut être introduit dans au moins une ouverture (22) à pratiquer dans l'au moins une région de fenestration (18).
